# EUROPEAN PATENT SPECIFICATION

(11) **EP 3 310 761 B1**
(45) Date of publication and mention of the grant of the patent: **05.10.2022**
(21) Application number: 15895847.0
(22) Date of filing: 17.12.2015
(51) Int. Cl.: C07D 215/50, C07D 239/48, C07D 241/42, C07D 403/12, A01N 1/02, C07D 241/40, C07D 209/04

(54) **PLATELET STORAGE METHODS AND COMPOSITIONS FOR SAME**
BLUTPLÄTTCHENSPEICHERVERFAHREN UND ZUSAMMENSETZUNGEN DAFÜR
PROCÉDÉS DE STOCKAGE DE PLAQUETTES ET COMPOSITIONS ASSOCIÉES

(30) Priority: 18.06.2015 US 201514743213
(43) Date of publication of application: 25.04.2018
(60) Divisional application: 22183075.5
(73) Proprietor: Platefuse, Inc., Cincinnati, OH 45249 (US)
(72) Inventor: ZHENG, Yi, Cincinnati, OH 45243 (US); CANCELAS, Jose, Cincinnati, OH 45249 (US)
(74) Representative: Samson & Partner Patentanwälte mbB
(86) International application number: PCT/US2015/066252
(87) International publication number: WO 2016/204809

(56) References cited:
- WO-A1-2013/166043
- US-A- 4 994 367
- US-A1- 2006 135 542
- US-A1- 2013 202 553
- US-A1- 2015 366 182
- M. LEVAY ET AL: "NSC23766, a Widely Used Inhibitor of Rac1 Activation, Additionally Acts as a Competitive Antagonist at Muscarinic Acetylcholine Receptors", JOURNAL OF PHARMACOLOGY AND EXPERIMENTAL THERAPEUTICS, vol. 347, no. 1, 25 July 2013 (2013-07-25) , pages 69-79, XP055530098, DOI: 10.1124/jpet.113.207266
- SHANG ET AL.: 'Rational design of small molecule inhibitors targeting RhoA subfamily Rho GTPases' CHEM BIOL. vol. 19, no. 6, 22 June 2012, pages 699 - 710, XP028497233
- AKBAR ET AL.: 'Gene Targeting Implicates Cdc42 GTPase in GPVI and Non-GPVI Mediated Platelet Filopodia Formation, Secretion and Aggregation' PLOS ONE vol. 6, no. 7, 2011, pages 1 - 9, XP055338333

## Description

### CROSS REFERENCE TO RELATED APPLICATIONS

This application claims priority to and benefit of US Patent Application Serial No. 14/743,213, filed June 18, 2015, of same title, in its entirety for all purposes.

### BACKGROUND

Patients with low platelet counts often require platelet transfusion. This is particularly crucial in the treatment of patients with cancer or massive trauma. The use of platelet transfusions has increased dramatically since 1980s, but a safe, long-term platelet storage method remains unavailable. The demonstration of successful, refrigerated storage of platelets for extended lengths of time, for example, 7 days or longer, would dramatically change the current practice of platelet transfusion in the Western World. Approximately 3,000,000 doses of platelets are used in the United States every year, and account for sales of ~$1.5 Billion annually. The current short shelf-life represents a major handicap to convert platelet products into effective commodities. Depending on the time of the year, month or even week, up to 20% of products can be wasted due to expiration. In the meantime, there are moments of platelets shortages due to unpredictable increased usage. The extension of platelet product shelf-life would strengthen the national inventory of platelets for oncological and trauma patients. An estimated 10-fold increase in the need of platelet and plasma products is expected by the US government in war casualties and massive trauma patients due to the 1 red cell:1 platelet: 1 plasma product transfusion policy.

Current practice has platelets stored at 20 to 24°C after preparation, which has a limited lifetime up to 5 days, primarily due to concerns about bacterial contamination. Bacterial contamination of platelet products for transfusion is a major safety problem in blood banking. The consequence of transfusion of contaminated products is increased morbi-mortality among a susceptible population of cancer patients (1). Different technologies have been developed aiming to minimize the risk of bacterial contamination including diversion pouches for collection, bacterial detection with automatic culture systems and pathogen reduction systems (2-6). While there has been a significant reduction in the number of cases of platelet transfusion associated sepsis, the risk of transfusion-associated sepsis ranges between 1 in 15,000 to 86,000 platelet transfusions (7, 8). Storage of platelets in cold temperatures, as is done for red cells, would reduce the proliferation of most bacteria and allow a longer period of storage (9), minimizing the current shortages (10) that the short storage time (5-day) for platelets approved by the FDA (11). Conventional cold storage of platelets, however, has been hampered by the discovery that the 24-hour recovery of chilled platelets was significantly reduced (14).

The development of a method to prevent platelet damage upon refrigeration is a much needed, and Jong sought after advance in blood banking. Such development would revolutionize the current method of platelet storage. The instant disclosure solves one or more of these deficiencies in the art.
US 4,994,367 discloses a composition for storage of blood platelets comprising an adenylate cyclase stimulator, a phosphodiesterase inhibitor, a thrombin, and a plasmin.

### BRIEF SUMMARY

Disclosed are compositions and methods for one or more of slowing, preventing, or reversing platelet damage, according to the claims, particularly as may occur during blood banking or during refrigeration of platelets. The composition include a RHOA inhibitor selected from: 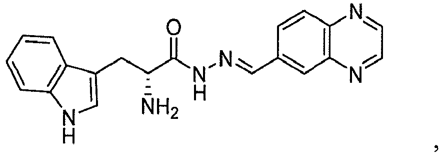 or salt thereof, 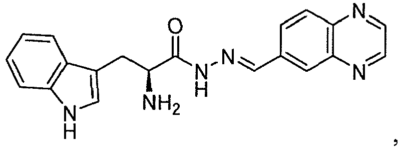 or salt thereof, or combinations thereof.. The compositions further includes a pharmaceutically acceptable carrier.

### BRIEF DESCRIPTION OF THE DRAWINGS

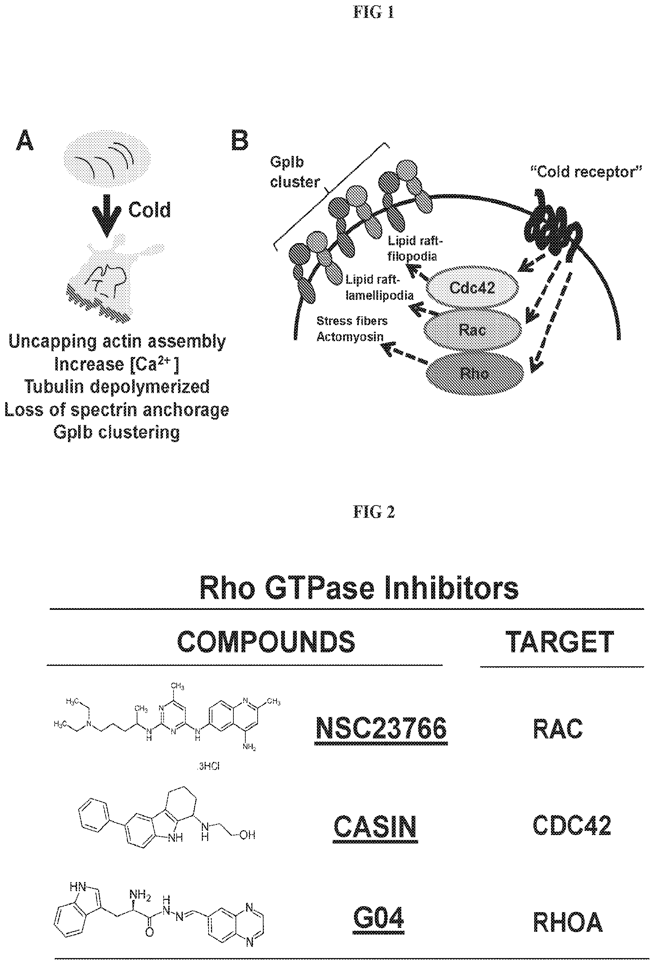

FIGS 1A-1B depict a schematic model of "cold receptor" initiated intracellular events involving Rho GTPases. 1A. Cold causes the actomyosin changes, Ca2+ mobilization, loss of spectrin anchorage, and Gplb clustering. 1B. "Cold receptor" may stimulate Cdc42, Rac, and/or Rho activation, which in turn control lipid raft assembly and actomyesin reorganization, resulting in Gplb clustering.
FIG 2 depicts Rho GTPases inhibitor chemical structures and targets.

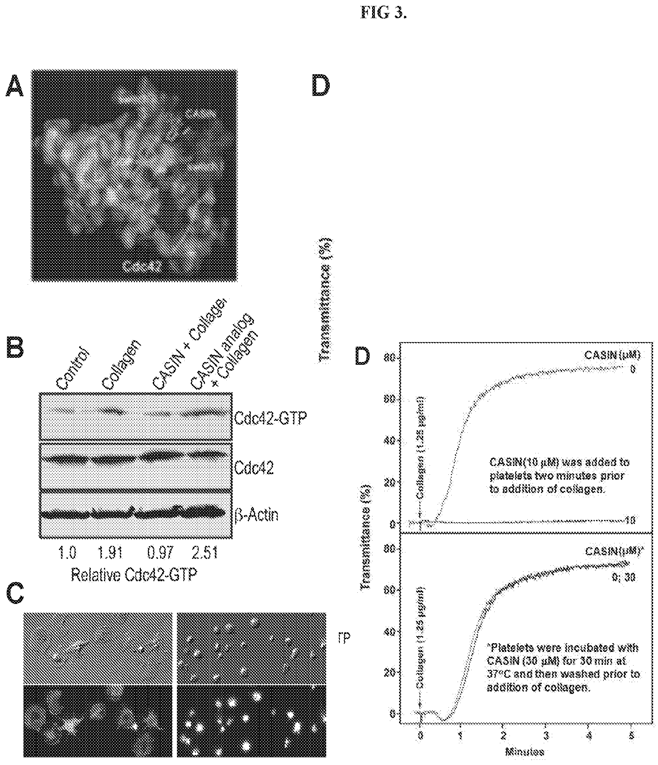

FIGS 3A-D depict a docking model of Casin bound to Cdc42 surface groove and data demonstrating Casin activity. 3A. Docking model of Casin bound to a Cdc42 surface groove required for activation. 3B. Collagen-induced Cdc42 activation is inhibited by Casin (10 µM) in a GST-PAK pulldown assay. 3C. Fibrinogen mediated platelet actin filopedia structure is blocked by Casin (10 µM). Rhodamine conjugated phalloidin-staining of platelets adhered to coated collagen surface. 3D. Collagen induced aggregation is blocked by 10 µM Casin (upper panel) and is reversible (up to 30 µM Casin) upon a wash of the inhibitor treated platelets (lower panel).

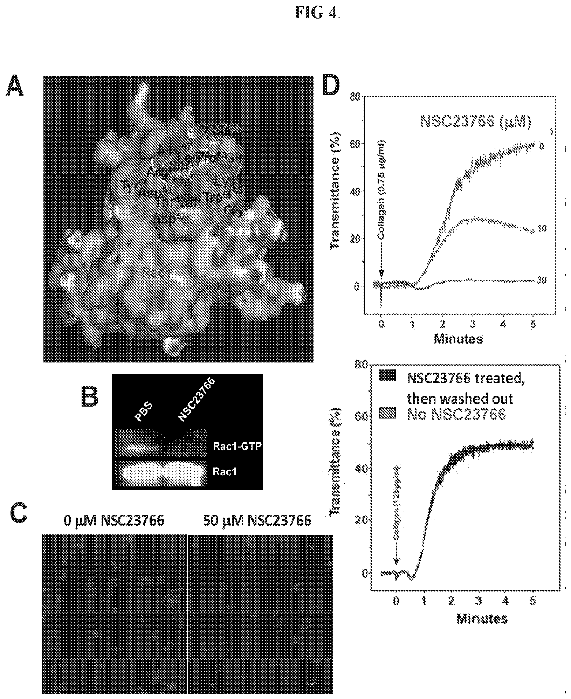

FIGS 4A-4E depict a docking model of Rac inhibitor NSC23766 and data showing NSC23766 activity. 4A. X-ray structure of NSC23766 bound to a Rac1 surface groove required for GEF activation. 4B. Rac1 activity is inhibited by NSC23766 (50 µM) in a GST-PAK pulldown assay. 4C. Fibrinogen-mediated platelet actin lamellopodia structure is blocked by NSC23766 (50 µM). Rhodamine conjugated phalloidin-staining of platelets adhered to coated collagen surface. 4D. Collagen induced aggregation is blocked by NSC23766 in a dose-dependent fashion. 4E. Inhibition of collagen induced platelet aggregation by NSC23766 (50µM) is reversible upon a wash of the inhibitor treated platelets.

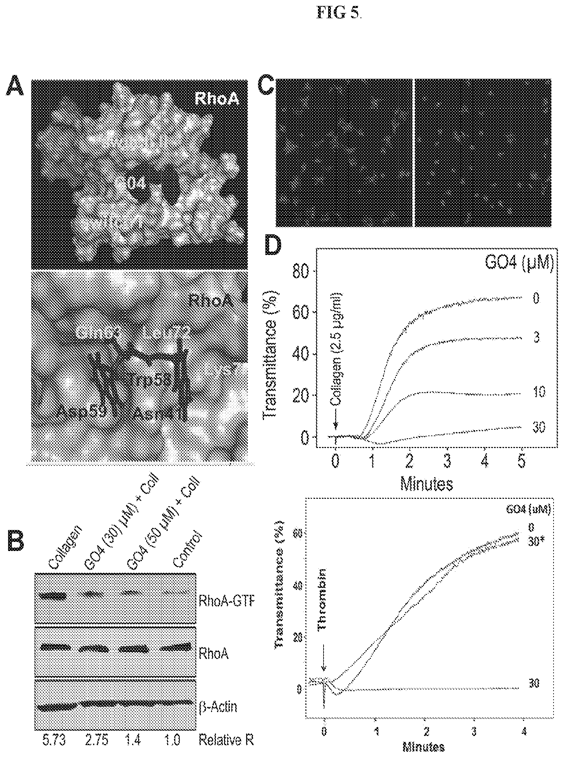

FIGS 5A-5D depict a docking model of Rho inhibitor G04 and data showing G04 activity. 5A. Docking model of G04 bound to a RhoA surface groove required for GEF activation. Upper panel: low resolution; Lower panel: high resolution binding domain. 5B. Collagen-induced RhoA activity is inhibited by Go4 (50 µM) in a GST-Rhotekin pulldown assay. 5C. U46629 (10 mM/Fibrinogen (3µM)-mediated platelet actin lamellopodia structure is blocked by G04 (30 µM). Rhodamine conjugated phalloidin-staining of platelets adhered to coated collagen surface. 5D. Collagen induced aggregation is inhibited by G04 (upper panel). Similar to collagen, thrombin induced aggregation (data not shown) is blocked by G04 in a dose-dependent fashion (upper panel) and is reversible upon a wash of the inhibitor treated platelets (lower panel).

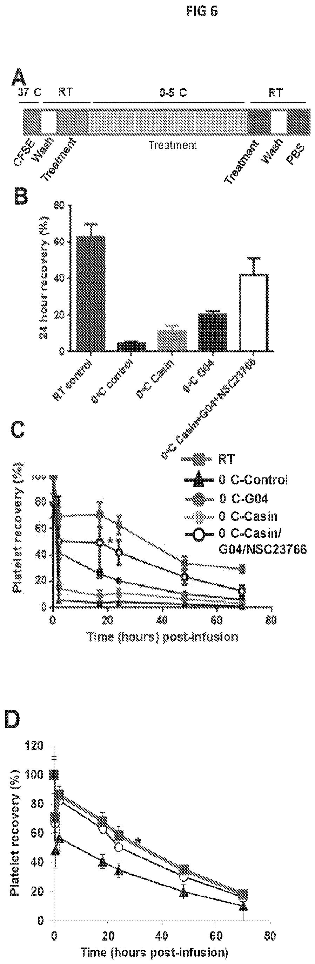

FIGS 6A-6D depict the effects of Rho GTPase inhibitor treatment on platelet transfusion. 6A. Experimental designs. 6B-6D. 24-hour recovery and survival of platelets in different conditions. 6B. 24-hour recovery. 6C-6D. Platelet recovery at different time points. Donor platelets were stored at room temperature (squares) or pretreated with a mixed Rho GTPase inhibitors (50 µM NSC23766, 10 µM CASIN and/or 75 µM G04) or no drug (control). Data are presented as mean SD. ^{∗}p<0.01 (Anova test with Bonferroni correction, between refrigerated with no inhibitor and refrigerated with triple inhibitor combination).

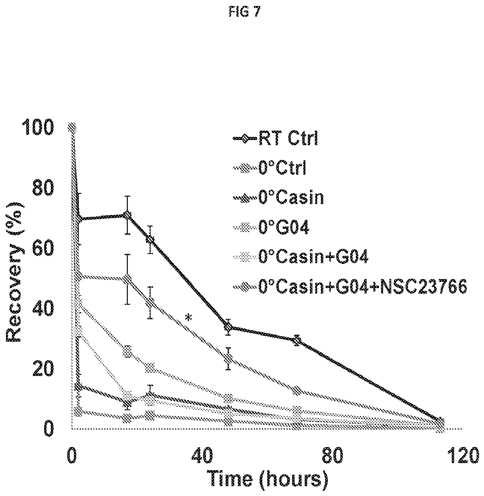

FIG 7 depicts effects of Rho GTPase inhibitor combinations on platelet survival upon transfusion. Platelet recovery at different time points is shown. Donor platelets were stored at room room temp (squares) or pretreated with a mixed Rho GPase inhibitors (50 uM NSC23766, 10 uM CASIN and/or 75 uM G04) or no drug (Ctrl). Data are presented as mean ±SD. ^{∗}p<0.01 (Anova test with Bonferroni correction, between refrigeratied with no inhibitor and refrigerated with triple inhibitor combination).

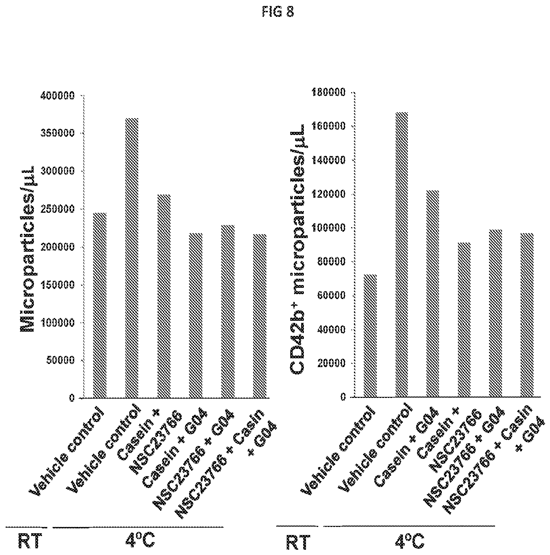

FIG 8 depicts total microparticle and G plb+ microparticle counts from platelets stored in different conditions. Results are representative of three independent experiments with similar results of platelets stored for 6 days in 50 µM NSC23766, 10 µM CASIN and/or 75 µM G04, or no drug (Vehicle control). The left panel shows microparticles/mcL of refrigerated stored platelets for 6 days; the right panel shows Gplb+ microparticle counts of refrigerated stored platelets for 6 days.

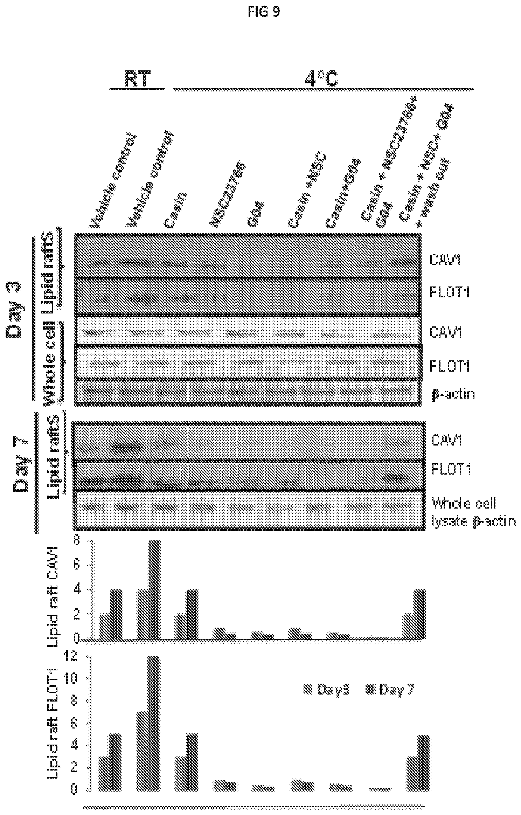

FIG 9 depicts amelioration of lipid raft formation by Rho GTPase inhibitor combinations. Platelets were stored at RT or 4°C for 3 or 7 days in different combinations of inhibitors (50 µM NSC23766, 10 µM CASIN and/or 75 µM G04) or no drug (vehicle control) at RT or 4°C. Platelet lysates were analyzed in the presence (lipid rafts) or absence (whole cell lysate) of Triton-X-100 mediated extraction. Results are representative of two independent experiments with similar results. Lowe panels represent normalized quantification of lipid raft formation.

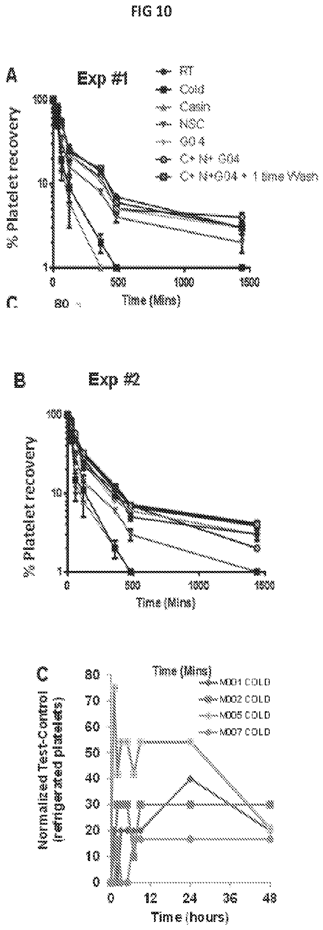

FIG 10A-10C depicts recovery (%) of human platelets transfused to pre-treated, thrombocytopenic NSG immunodevicient mice (FIG 10A and 10B); FIG C shows differential recovery between test and control in a randomized, crossover trial of treated refrigerated platelets in atologous transfusion of 7-day stored Rhesus monkey platelets.

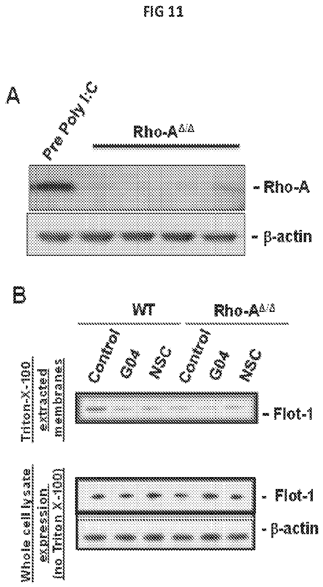

FIG 11A-11B depicts RhoA deletion (RhoA^{Δ/Δ}) after administratoin of poly I:C compared with pre- poly I:C RhoA expresion levels in platelets. FIG 11B depicts Flotillin-1 and actin expression in lipid rafts (upper panel) and in whole platelet lysates (lower panel).

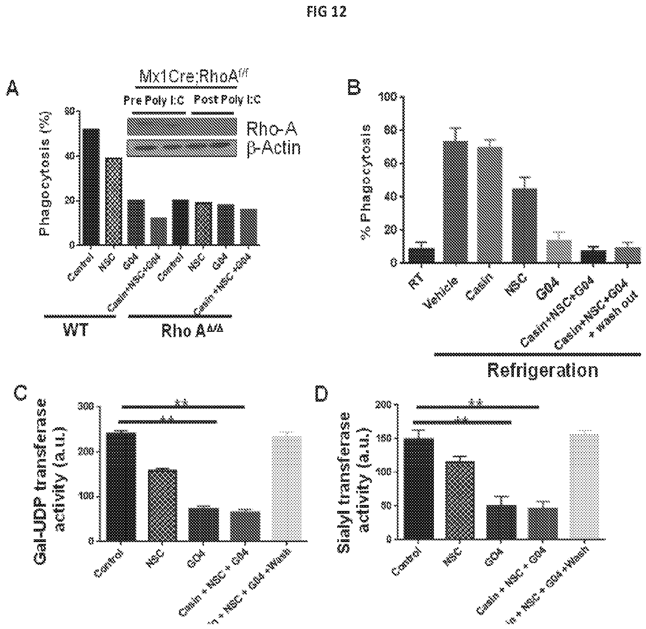

FIG 12A-12D depict the effect of Rho family inhibitors on murine wild-type and RhoA-deficient platelets (12A); the effect of Rho family inhibitors on human platelets (12B); and the effect of Rho family inhibitors on galactosyl- (12C) and sialyl- (12D) transferase activities to the platelet membranes.

### DETAILED DESCRIPTION

Unless defined otherwise, all technical and scientific terms used herein have the same meaning as commonly understood by one of ordinary skill in the art to which the embodiments belong.

### DEFINITIONS

It must be noted that as used herein and in the appended claims, the singular forms "a," "an," and "the" include plural referents unless the context clearly dictates otherwise. Thus, for example, reference to "a platelet" includes a plurality of such platelets and reference to "the carrier" includes reference to one or more carriers and equivalents thereof known to those skilled in the art, and so forth.

The term "about" or "approximately" means within an acceptable error range for the particular value as determined by one of ordinary skill in the art, which will depend in part an how the value is measured or determined, e.g., the limitations of the measurement system. For example, "about" can mean within 1 or more than 1 standard deviations, per the practice in the art. Where particular values are described in the application and claims, unless otherwise stated the term "about" meaning within an acceptable error range for the particular value should be assumed.

An "amide" is a chemical moiety with formula -(R)n-C(0)NHR' or -(R)n-NHC(0)R', where R and R' are independently selected from the group consisting of alkyl, cycloalkyl, aryl, heteroaryl (bonded through a ring carbon) and heteroalicyclic (bonded through a ring carbon), and where n is 0 or 1.

The term "aromatic" refers to an aromatic group which has at least one ring having a conjugated pi electron system and includes both carbocyclic aryl (e.g., phenyl) and heterocyclic aryl groups (e.g., pyridine). The term includes monocyclic or fused-ring polycyclic (i.e., rings which share adjacent pairs of carbon atoms) groups. The term "carbocyclic" refers to a compound which contains one or more covalently closed ring structures, and that the atoms forming the backbone of the ring are all carbon atoms. The term thus distinguishes carbocyclic from heterocyclic rings in which the ring backbone contains at least one atom which is different from carbon. The term "heteroaromatic" refers to an aromatic group which contains at least one heterocyclic ring.

As used herein, the term "alkyl" refers to an aliphatic hydrocarbon group. The alkyl moiety may be a "saturated alkyl" group, which means that it does not contain any alkene or alkyne moieties. The alkyl moiety may also be an "unsaturated alkyl" moiety, which means that it contains at least one alkene or alkyne moiety. An "alkene" moiety refers to a group consisting of at least two carbon atoms and at least one carbon-carbon double bond, and an "alkyne" moiety refers to a group consisting of at least two carbon atoms and at least one carbon-carbon triple bond. The alkyl moiety, whether saturated or unsaturated, may be branched, straight chain, or cyclic.

The alkyl group may have 1 to 20 carbon atoms (whenever it appears herein, a numerical range such as "1 to 20" refers to each integer in the given range; e.g., "1 to 20 carbon atoms" means that the alkyl group may consist of 1 carbon atom, 2 carbon atoms, 3 carbon atoms, etc., up to and including 20 carbon atoms, although the present definition also covers the occurrence of the term "alkyl" where no numerical range is designated). The alkyl group may also be a medium size alkyl having 1 to 10 carbon atoms. The alkyl group could also be a lower alkyl having 1 to 5 carbon atoms. The alkyl group of the compounds of the invention may be designated as "C1-C4 alkyl" or similar designations. By way of example only, "C1-C4 alkyl" indicates that there are one to four carbon atoms in the alkyl chain, i.e., the alkyl chain is selected from the group consisting of methyl, ethyl, propyl, iso-propyl, n-butyl, iso-butyl, sec-butyl, and t-butyl.

The alkyl group may be substituted or unsubstituted. When substituted, the substituent group(s) is(are) one or more group(s) individually and independently selected from cycloalkyl, aryl, heteroaryl, heteroalicyclic, hydroxy, alkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, S-sulfonamido, N-sulfonamido, C-carboxy, O-carboxy, isocyanato, thiocyanato, isothiocyanato, nitro, silyl, trihalomethanesulfonyl, and amino, including mono- and di-substituted amino groups, and the protected derivatives thereof. Typical alkyl groups include, but are in no way limited to, methyl, ethyl, propyl, isopropyl, butyl, isobutyl, tertiary butyl, pentyl, hexyl, ethenyl, propenyl, butenyl, cyclopropyl, cyclobutyl, cyclopentyl, cyclohexyl, and the like. Wherever a substituent is described as being "optionally substituted" that substitutent may be substituted with one of the above substituents.

The term "ester" refers to a chemical moiety with formula -(R)n-COOR', where R and R' are independently selected from the group consisting of alkyl, cycloalkyl, aryl, heteroaryl (bonded through a ring carbon) and heteroalicyclic (bonded through a ring carbon), and where n is 0 or 1.

The substituent "R" appearing by itself and without a number designation refers to a substituent selected from the group consisting of hydrogen, alkyl, cycloalkyl, aryl, heteroaryl (bonded through a ring carbon) and heteroalicyclic (bonded through a ring carbon).

An "O-carboxy" group refers to a RC(=O)O- group, where R is as defined herein.

A "C-carboxy" group refers to a -C(=O)OR groups where R is as defined herein.

An "acetyl" group refers to a -C(=O)CH3, group.

A "trihalomethanesulfonyl" group refers to a X3CS(=O)2- group where X is a halogen.

A "cyano" group refers to a -CN group.

An "isocyanato" group refers to a -NCO group.

A "thiocyanato" group refers to a -CNS group.

An "isothiocyanato" group refers to a -NCS group.

A "sulfinyl" group refers to a -S(=O)-R group, with R as defined herein.

A "S-sulfonamido" group refers to a -S(=O)2NR, group, with R as defined herein.

A "N-sulfonamido" group refers to a RS(=O)2NH- group with R as defined herein.

A "trihalomethanesulfonamido" group refers to a X3CS(=O)2NR-group with X and R as defined herein.

An "O-carbamyl" group refers to a -OC(=O)-N(R)2, group-with R as defined herein.

An "N-carbamyl" group refers to a ROC(=O)NH- group, with R as defined herein.

An "O-thiocarbamyl" group refers to a -OC(=S)-N(R)2, group with R as defined herein.

An "N-thiocarbamyl" group refers to an ROC(=S)NH- group, with R as defined herein.

A "C-amido" group refers to a -C(=O)-N(R)2 group with R as defined herein.

An "N-amido" group refers to a RC(=O)NH- group, with R as defined herein.

The term "perhaloalkyl" refers to an alkyl group where all of the hydrogen atoms are replaced by halogen atoms.

The term "acylalkyl" refers to a RC(=O)R'- group, with R as defined herein, and R' being a diradical alkylene group. Examples of acylalkyl, without limitation, may include CH3C(=O)CH2-, CH3C(=O)CH2CH2-, CH3CH2C(=O)CH2CH2-, CH3C(=O)CH2CH2CH2-, and the like.

Unless otherwise indicated, when a substituent is deemed to be "optionally substituted," it is meant that the substitutent is a group that may be substituted with one or more group(s) individually and independently selected from cycloalkyl, aryl, heteroaryl, heteroalicyclic, hydroxy, alkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, S-sulfonamido, N-sulfonamido, C-carboxy, O-carboxy, isocyanato, thiocyanato, isothiocyanato, nitro, silyl, trihalomethanesulfonyl, and amino, including mono- and di-substituted amino groups, and the protected derivatives thereof.

The terms "protecting group" and "protecting groups" as used herein refer to any atom or group of atoms that is added to a molecule in order to prevent existing groups in the molecule from undergoing unwanted chemical reactions. The protecting group moiety may be chosen in such a way, that they are stable to the reaction conditions applied and readily removed at a convenient stage using methodology known from the art. A non-limiting list of protecting groups include benzyl; substituted benzyl; alkylcarbonyls (e.g., t-butoxycarbonyl (BOC)); arylalkylcarbonyls (e.g., benzyloxycarbonyl, benzoyl); substituted methyl ether (e.g. methoxymethyl ether); substituted ethyl ether; a substituted benzyl ether; tetrahydropyranyl ether; silyl ethers (e.g., trimethylsilyl, triethylsilyl, triisopropylsilyl, t-butyldimethylsilyl, or t-butyldiphenylsilyl); esters (e.g. benzoate ester); carbonates (e.g. methoxymethylcarbonate); sulfonates (e.g. tosylate, mesylate); acyclic ketal (e.g. dimethyl acetal); cyclic ketals (e.g., 1,3-dioxane or 1,3-dioxolanes); acyclic acetal; cyclic acetal; acyclic hemiacetal; cyclic hemiacetal; and cyclic dithioketals (e.g., 1,3-dithiane or 1,3-dithiolane).

As used herein, the term "cycloalkyl" is intended to cover three-, four-, five-, six-, seven-, and eight- or more membered rings comprising carbon atoms only. A cycloalkyl can optionally contain one or more unsaturated bonds situated in such a way, however, that an aromatic pi-electron system does not arise. Some examples of "cycloalkyl" are the carbocycles cyclopropane, cyclobutane, cyclopentane, cyclopentene, cyclopentadiene, cyclohexane, cyclohexene, 1,3-cyclohexadiene, 1,4-cyclohexadiene, cycloheptane, or cycloheptene.

As used herein, "heterocyclyl" means a cyclic ring system comprising at least one heteroatom in the ring system backbone. The heteroatoms are independently selected from oxygen, sulfur, and nitrogen. Heterocyclyls may include multiple fused rings. Heterocyclyls may have any degree of saturation provided that at least one ring in the ring system is not aromatic. Heterocyclyls may be substituted or unsubstituted, and are attached to other groups via any available valence, preferably any available carbon or nitrogen. Preferred monocyclic heterocycles are of 5 or 6 members. In six membered monocyclic heterocycles, the heteroatom(s) are selected from one up to three of oxygen, sulfur, and nitrogen, and wherein when the heterocycle is five membered, preferably it has one or two heteroatoms selected from oxygen, sulfur, and nitrogen.

A heterocyclyl can further contain one or more carbonyl or thiocarbonyl functionalities, so as to make the definition include oxo-systems and thio-systems such as lactams, lactones, cyclic imides, cyclic thioimides, cyclic carbamates, and the like. Some examples of "heterocyclyls" include, but are not limited to, tetrahydrothiopyran, 4H-pyran, tetrahydropyran, piperidine, 1,3-dioxin, 1,3-dioxane, 1,4-dioxin, 1,4-dioxane, piperazine, 1,3-oxathiane, 1,4-oxathiin, 1,4-oxathiane, tetrahydro-1,4-thiazine, 2H-1,2-oxazine, maleimide, succinimide, barbituric acid, thiobarbituric acid, dioxopiperazine, hydantoin, dihydrouracil, morpholine, trioxane, hexahydro-1,3,5-triazine, tetrahydrothiophene, tetrahydrofuran, pyrroline, pyrrolidine, pyrrolidone, pyrrolidione, pyrazoline, pyrazolidine, imidazoline, imidazolidine, 1,3-dioxole, 1,3-dioxolane, 1,3-dithiole, 1,3-dithiolane, isoxazoline, isoxazolidine, oxazoline, oxazolidine, oxazolidinone, thiazoline, thiazolidine, and 1,3-oxathiolane. The attachment point of a heterocycle radical can be at the position of a nitrogen heteroatom or via a carbon atom of the heterocycle.

The term "aryl" means a carbocyclic aromatic ring or ring system. Moreover, the term "aryl" includes fused ring systems wherein at least two aryl rings, or at least one aryl and at least one C3-8-cycloalkyl share at least one chemical bond. Some examples of "aryl" rings include optionally substituted phenyl, naphthalenyl, phenanthrenyl, anthracenyl, tetralinyl, fluorenyl, indenyl, and indanyl. The term "aryl" relates to aromatic, including, for example, benzenoid groups, connected via one of the ring-forming carbon atoms, and optionally carrying one or more substituents selected from heterocyclyl, heteroaryl, halo, hydroxy, amino, cyano, nitro, alkylamido, acyl, C1-6 alkoxy, C1-6 alkyl, C1-6 hydroxyalkyl, C1-6 aminoalkyl, C1-6 alkylamino, alkylsulfenyl, alkylsulfinyl, alkylsulfonyl, sulfamoyl, or trifluoromethyl. The aryl group can be substituted at the para and/or meta positions. In other embodiments, the aryl group can be substituted at the ortho position. Representative examples of aryl groups include, but are not limited to, phenyl, 3-halophenyl, 4-halophenyl, 3-hydroxyphenyl, 4-hydroxyphenyl, 3-aminophenyl, 4-aminophenyl, 3-methylphenyl, 4-methylphenyl, 3-methoxyphenyl, 4-methoxyphenyl, 4-trifluoromethoxyphenyl 3-cyanophenyl, 4-cyanophenyl, dimethylphenyl, naphthyl, hydroxynaphthyl, hydroxymethylphenyl, trifluoromethylphenyl, alkoxyphenyl, 4-morpholin-4-yl-phenyl, 4-pyrrolidin-1-ylphenyl, 4-pyrazolylphenyl, 4-triazolylphenyl, and 4-(2-oxopyrrolidin-1-yl)phenyl.

As used herein, the term "heteroaryl" means an aromatic radical having one or more heteroatom(s) (e.g., oxygen, sulfur, or nitrogen) in the ring backbone and may include a single ring (e.g., pyridine) or multiple condensed rings (e.g., quinoline). Heteroaryl groups can carry one or more substituents, each independently selected from halo, hydroxy, amino, cyano, nitro, cycloalkyl, haloalkyl, aryl, heterocyclyl, mercapto, alkylamido, acyl, C1-6-alkoxy, C1-6-alkyl, C1-6-hydroxyalkyl, C1-6-aminoalkyl, C1-6-alkylamino, alkylsulfenyl, alkylsulfinyl, alkylsulfonyl, sulfamoyl, and trifluoromethyl. Representative examples of heteroaryl groups include, but are not limited to, optionally substituted derivatives of furan, benzofuran, thiophene, benzothiophene, pyrrole, pyridine, indole, oxazole, benzoxazole, isoxazole, benzisoxazole, thiazole, benzothiazole, isothiazole, imidazole, benzimidazole, pyrazole, indazole, tetrazole, quionoline, isoquinoline, pyridazine, pyrimidine, purine and pyrazine, furazan, 1,2,3-oxadiazole, 1,2,3-thiadiazole, 1,2,4-thiadiazole, triazole, benzotriazole, pteridine, phenoxazole, oxadiazole, benzopyrazole, quinolizine, cinnoline, phthalazine, quinazoline, and quinoxaline. In some embodiments, the substituents can be halo, hydroxy, cyano, O-C1-6-alkyl, C1-6-alkyl, hydroxy-C1-6-alkyl, and amino-C1-6-alkyl.

The term "platelet" is used here to refer to a blood platelet. A platelet can be described as a minisule protoplasmic disk occurring in vertebrate blood. Platelets play a role in blood clotting. The platelet may be derived from any source including a human blood supply, or the patient's own blood.

As used herein, the term "effective amount" means the amount of one or more active components that is sufficient to show a desired effect. This includes both therapeutic and prophylactic effects. When applied to an individual active ingredient, administered alone, the term refers to that ingredient alone. When applied to a combination, the term refers to combined amounts of the active ingredients that result in the therapeutic effect, whether administered in combination, serially or simultaneously.

Refrigerated storage is believed to reduce platelet life-span due to decreased temperature that cause glycoprotein-Ib (GPIb) receptors to cluster on specific microdomains of the platelet membrane. Applicant has found that recognition of specific glycated/syalylated residues on clustered glycoproteins by macrophage β2 integrins and hepatocyte Ashwell-Morell receptors results in platelet phagocytosis by the host and removal from circulation. Thus, Applicant has identified prevention of glycoprotein clustering as a useful target for chemical intervention.

Platelet glycoproteins are intimately associated with intracellular cytoskeleton. Clustering of platelet glycoproteins depends on the formation of lipid raft in the platelet membrane, which in turn depends on the dynamics of the highly regulated processes of actomyosin assembly/disassembly. Rho family GTPases, including RhoA, Rac1 and Cdc42, are a class of GTP-binding enzymes that are central regulators of F-actin polymerization/depolymerization, and have been shown to control lipid raft formation and composition. Therefore, Applicant postulates that changes in Rho GTPase activities may influence platelet membrane lipid raft assembly and glycoprotein composition. Reversible targeting of Rho family GTPases by small molecule inhibitors may prevent cytoskeleton-dependent refrigeration storage lesions in platelets and result in increased platelet survival.

The mechanisms of how cold temperatures affect platelet survival are not completely understood, though significant information has been collected in the past decade. The effects of cold temperature on platelets are believed to be complex and involve shape change, cytoskeletal reorganization, activation, cell surface protein clustering and changes in the carbohydrate structures of surface glycoproteins (15-18). Refrigeration-induced changes including filopodia or lamellipodia are accompanied by an increase in the fraction of total cellular actin in a polymeric state (F-actin) (12, 18, 19) and disappearance of a peripheral microtubule coil (20). Isolated prevention of microtubule polymerization using colchicine has not resulted in shape change prevention upon activation (21, 22). Prevention of isolated actin dynamics using cytochalasin B results in reversion of discoid shape (18) but not in improved platelet survival in baboons (23), suggesting that irreversible blockade of actin polymerization does not prevent the refrigeration damage.

Presence of platelet cold receptors has been postulated as an explanation for both homeostatic and clinical effects when platelets are submitted to temperatures below 16°C. Cold temperature is believed to induce deglycosylation of glycoprotein Ib ectodomain exposing N-acetyl-D-glucosamine residues (17), which sequesters GM1 gangliosides in lipid rafts. Raft-associated glycoprotein Iba forms clusters upon binding of 14-3-3z adaptor proteins to its cytoplasmic tail, a process accompanied with mitochondrial damage and PS exposure (apoptosis-like)(24). The mechanisms of platelet clearance are believed to be associated with lipid-raft associated GPIb clustering and prevention of clustering prevents platelet clearance (15, 25). Intimately associated with intracellular cytoskeleton, GPIb clustering depends on the formation of microdomains (so-called "lipid rafts") in the platelet membrane which in turn depends on the dynamics of the highly regulated processes of acto-myosin assembly/disassembly at multiple levels. In summary, refrigeration results in multiple, complex platelet defects that may be closely associated with cytoskeletal impairments at multiple levels (FIG 1A).

Actin cytoskeletal rearrangements responsible for lipid rafts and GPIb clustering in lipid rafts depends on the coordinated activities of Cdc42, Rac1 and RhoA GTPases, which control specific downstream effectors in regulating polymerization and depoymerization of F-actin, actomyosin contraction, tubulin polymerization, and spectrin anchorage. The Rho family GTPases are a class of GTP-binding enzymes that act as signaling switches in spatial/temporal transduction and amplification of signals from platelet receptors to the intracellular signaling pathways that drive platelet function. Among the direct Rho GTPase effectors, WASPs, formins and PAKs that control F-actin polymerization/depolymerization have been shown to be crucial in the control of lipid raft formation and composition and tubular polymerization of platelets (27) (FIG 1B). Therefore, changes in Rho GTPase activities may influence platelet membrane microdomain assembly and glycoprotein composition. Earlier studies using dominant negative mutants of Cdc42 and Rad 1 found no effect an prevention of cold-induced platelet damage (28), but the limitation of the tools used has prevented investigators from manipulating actin/actomyosin dynamics in a specific, reversible fashion.

Without intending to be limited by theory, it is believed that reversible inhibition of multiple Rho family of GTPases by chemical inhibitors can significantly improve platelet survival and transfusion function after refrigerated storage by interference with actomyosin dynamics and membrane microdomains to prevent GPIb clustering.

Compositions and methods useful for platelet survival and/or quality, transfusion, and associated issues are disclosed herein. In one aspect, a composition for platelet storage or treatment is described.

The RHOA inhibitor comprises (Formula III)(G04), or a pharmaceutically acceptable salt thereof.

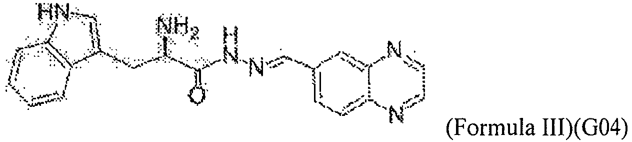

The active agent can form salts. Reference to a compound of the active agent herein is understood to include reference to salts thereof, unless otherwise indicated. The term "salt(s)", as employed herein, denotes acidic and/or basic salts formed with inorganic and/or organic acids and bases. In addition, when an active agent contains both a basic moiety, such as, but not limited to an amine or a pyridine or imidazole ring, and an acidic moiety, such as, but not limited to a carboxylic acid, zwitterions ("inner salts") can be formed and are included within the term "salt(s)" as used herein. Pharmaceutically acceptable (e.g., nontoxic, physiologically acceptable) salts are preferred, although other salts are also useful, e.g., in isolation or purification steps, which can be employed during preparation. Salts of the compounds of the active agent can be formed, for example, by reacting a compound of the active agent with an amount of acid or base, such as an equivalent amount, in a medium such as one in which the salt precipitates or in an aqueous medium followed by lyophilization.

The active agents which contain a basic moiety, such as, but not limited to an amine or a pyridine or imidazole ring, can form salts with a variety of organic and inorganic acids. Exemplary acid addition salts include acetates (such as those formed with acetic acid or trihaloacetic acid, for example, trifluoroacetic acid), adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides (formed with hydrochloric acid), hydrobromides (formed with hydrogen bromide), hydroiodides, 2-hydroxyethanesulfonates, lactates, maleates (formed with maleic acid), methanesulfonates (formed with methanesulfonic acid), 2-naphthalenesulfonates, nicotinates, nitrates, oxalates, pectinates, persulfates, 3-phenylpropionates, phosphates, picrates, pivalates, propionates, salicylates, succinates, sulfates (such as those formed with sulfuric acid), sulfonates (such as those mentioned herein), tartrates, thiocyanates, toluenesulfonates such as tosylates, undecanoates, and the like.

The active agents that contain an acidic moiety, such as, but not limited to a carboxylic acid, may form salts with a variety of organic and inorganic bases. Exemplary basic salts include ammonium salts, alkali metal salts such as sodium, lithium, and potassium salts, alkaline earth metal salts such as calcium and magnesium salts, salts with organic bases (for example, organic amines) such as benzathines, dicyclohexylamines, hydrabamines [formed with N,N-bis(dehydro-abietyl)ethylenediamine], N-methyl-D-glucamines, N-methyl-D-glucamides, t-butyl amines, and salts with amino acids such as arginine, lysine and the like. Basic nitrogen-containing groups can be quaternized with agents such as lower alkyl halides (e.g., methyl, ethyl, propyl, and butyl chlorides, bromides and iodides), dialkyl sulfates (e.g., dimethyl, diethyl, dibutyl, and diamyl sulfates), long chain halides (e.g., decyl, lauryl, myristyl and stearyl chlorides, bromides and iodides), aralkyl halides (e.g., benzyl and phenethyl bromides), and others.

Active agent, and salts thereof, can exist in their tautomeric form (for example, as an amide or imino ether).

All stereoisomers of the present compounds, such as those, for example, which can exist due to asymmetric carbons on any of the substituents, including enantiomeric forms (which can exist even in the absence of asymmetric carbons) and diastereomeric forms, are contemplated and within the scope of the preferred embodiments. Individual stereoisomers of the compounds of the preferred embodiments can, for example, be substantially free of other isomers, or can be admixed, for example, as racemates or with all other or other selected, stereoisomers. The chiral centers of the preferred embodiments can have the S or R configuration as defined by the IUPAC 1974 Recommendations.

When the compounds are in the forms of salts, they may comprise pharmaceutically acceptable salts. Such salts may include pharmaceutically acceptable acid addition salts, pharmaceutically acceptable base addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, sulphates, nitrates, phosphates, perchlorates, borates, acetates, benzoates, hydroxynaphthoates, glycerophosphates, ketoglutarates and the like. Examples of metal salts include lithium, sodium, potassium, magnesium salts and the like. Examples of ammonium and alkylated ammonium salts include ammonium, methylammonium, dimethylammonium, trimethylammonium, ethylammonium, hydroxyethylammonium, diethylammonium, butylammonium, tetramethylammonium salts and the like. Examples of organic bases include lysine, arginine, guanidine, diethanolamine, choline and the like.

The pharmaceutically acceptable salts may be prepared by methods known to one of ordinary skill in the art. For example, by reacting the active agent with 1 to 4 equivalents of a base such as sodium hydroxide, sodium methoxide, sodium hydride, potassium t-butoxide, calcium hydroxide, magnesium hydroxide and the like, in solvents like ether, THF, methanol, t-butanol, dioxane, isopropanol, ethanol, etc. Mixture of solvents can be used. Organic bases like lysine, arginine, diethanolamine, choline, guandine and their derivatives etc. can also be used. Alternatively, acid addition salts wherever applicable are prepared by treatment with acids such as hydrochloric acid, hydrobromic acid, nitric acid, sulfuric acid, phosphoric acid, p-toluenesulphonic acid, methanesulfonic acid, fonic acid, acetic acid, citric acid, maleic acid salicylic acid, hydroxynaphthoic acid, ascorbic acid, palmitic acid, succinic acid, benzoic acid, benzenesulfonic acid, tartaric acid and the like in solvents like ethyl acetate, ether, alcohols, acetone, THF, dioxane, etc. Mixture of solvents can also be used.

The above-described compositions may comprise a physiologically acceptable carrier. In one aspect, the physiologically acceptable carrier may comprise a buffer. In one aspect, the physiologically acceptable carrier may be selected from saline, phosphate buffered saline, Tris buffered saline, Hank's buffered saline, water, or a combination thereof. In one aspect, the physiologically acceptable carrier may comprise an electrolyte solution.

In one aspect, the RHOA inhibitor may be present in a carrier at a concentration, in combination
or separately, of at least about 10 µM or at least about 25 µM at least about 50 µM at least about 100 µM, at least about 200 µM, at least about 500 µM, at least about 1 mM, at least about 10 mM, at least about 50 mM, at least about 100 mM, or at least about 1 M.

In one aspect the RHOA inhibitor may be present at a concentration of from about 10 µM to about 500 µM, or from about 25 µM to about 400 µM, or from about 50 µM to about 300 µM, or from about 75 µM to about 200 µM, or from about 100 µM to about 150 µM, or about 75 gm.

In further aspects, the described compositions may comprise an additive selected from NaCl, KC1, CaC12, MgCl₂, MgSO₄, Na³ citrate, citric acid, NaHCO₃, Na phosphate, Na acetate, Na gluconate, glucose, maltose, mannitol, and combinations thereof. The described compositions may comprise an additive selected from NaCl, KC1, CaCl₂, MgCl₂, MgSO₄, Na³ citrate, citric acid, NaHCO₃, Na phosphate, Na acetate, Na gluconate, glucose, maltose, mannitol, and combinations thereof, wherein said additive may be present in an amount of from about 0.5 mmol/L to about 150 mmol/L.

In further aspects, the described compositions may comprise one or more ingredients selected from D-ribose, D-glucose, Hanks solution, Hepes solution, bovine serum albumin, tic anticoagulant peptide and sterile water, or combinations thereof.

In one aspect, the composition may comprise an auxiliary substance selected from pH adjusting and buffering agents, tonicity adjusting agents, stabilizers, wetting agents, and combinations thereof.

In one aspect, the composition may have a pH of from about 5 to about 8, or from about 6 to about 7, or about 6.8 to about 7.4.

In one aspect, the composition may be isotonic.

In yet further aspects, the composition may comprise an additional therapeutic agent.

A method for storing platelets is also described. In this aspect, the method comprises the step of storing platelets in a composition as disclosed herein.

In one aspect, the method may comprise contacting platelets with a solution comprising a
RHOA inhibitor, wherein the one or more actives are present in a carrier at a concentration, in combination or separately, of at least about 10 µM, or at least about 25 µM, at least about 50 µM, at least about 1001.1M, at least about 2001.1M, at least about 5001.1M, at least about 1 mM, at least about 10 mM, at least about 50 mM, at least about 100 mM, or at least about 1 M.

In one aspect, the method may comprise contacting platelets with a solution comprising an RHOA inhibitor present in a solution at a concentration of from about 10 µM to about 5001.1M, or from about 25 1.IM to about 4001.1M, or from about 501.IM to about 3001.1M, or from about 75 µM to about 200 µM, or from about 100 µM to about 150 µM, or about 75 µM.

In one aspect, the storage step may be carried out at a temperature of from about 1°C to about 20°C, or at about 1°C, or about 2°C, or about 3°C, or about 4°C, or about 5°C, or about 6°C, or about 7°C, or about 8°C, or about 9°C, or about 10°C, or about 11°C, or about 12°C, or about 13°C, or about 14°C, or about 15°C, or about 16°C.

Ine aspect, the storage step may be carried out for a period of time of from about 7 to 20 days, or from about 10 to 15 days, or greater than 7 days, or greater than 8 days, or greater than 9 days, or greater than 10 days, or greater than 11 days, or greater than 12 days, or greater than 13 days, or greater than two weeks.

In one asepct, the composition may be used in an amount sufficient to inhibit a platelet damaging activity selected from polymerization of F-actin, depolymerization of F-actin, actomyosin contraction, tubulin polymerization, spectrin anchorage, or combinations thereof.

In one aspect, applying the described methods, the platelet survival may be greater than about 65%, or greater than about 70%, or greater than about 75%, or greater than about 80%, or greater than about 85%, or greater than about 90%, or greater than about 95%, or greater than about 99% after a storage period of 24 hours at 5C.

In one aspect, a method of reversing platelet activation is also disclosed, comprising contacting activated platelets with a composition as described herein. In one aspect, the contacting step may be carried out at a temperature of about 0°C. In a yet further aspect, the disclosed compositions may be used to reverse refrigeration storage lesions in platelets. In this aspect, platelets having refrigeration storage lesions may be contacted with a composition as disclosed herein, for a period of time sufficient to reverse refrigeration storage lesions.

### Examples including reference examples

Applicant has tested the ability of three chemical inhibitors, CASIN, NSC23766 and G04, targeting Cdc42, Rad and RhoA, respectively (FIG 2), to inhibit the activity of the GTPases and the consequent cytoskeleton-dependent functions of human platelets. CASIN and NSC23766 are unclaimed reference examples referred to in the examples of this application. A rationally developed inhibitor of Cdc42 with activity on activation by guanine nucleotide exchange factors is CASIN (29, *Nature Biotech* under revision). CASIN was recently discovered by Zheng group as a small molecule that recognizes a pocket domain that specifically blocks the ability of GEF interaction with Cdc42 (FIG 3A). CASIN is found to suppress of hematopoietic stem cell aging through its specific Cdc42 inhibitory activity (29). In platelets, it inhibits Cdc42 activation (FIG 3B) and prevents collagen-induced platelet shape changes (FIG 3C). These changes depend on integrin signaling and involve F-actin polymerization and filopodia formation as demonstrated by phalloiding staining (FIG 3C, lower panels). Cdc42 inhibition by CASIN results in prevention of collagen-induced platelet aggregation (FIG 3D) that can be reversed by a washout of the inhibitor (FIG 3D, upper and lower panels).

Similarly, an inhibitor of Rac, NSC23766 (3034), with an ability to impair Rac 1 activation by multiple activating signals which may be crucial in platelet signaling associated with Gplb/GpX (35). NSC23766 was discovered in a structure-based virtual screening of compounds that fit into a surface groove of Rac 1 known to be critical for guanine nucleotide exchange factor specification (34) (FIG 4A). NSC23766 can effectively block Rac 1 activation (FIG 4B) and Rac-dependent cytoskeletal rearrangements (actin lamellopodia) of platelets stimulated by collagen (30, 32, 35), indicating its ability for suppressing collagen-integrin dependent signaling (FIG 4C). Finally, NSC23766 reversibly inhibits, in a dose-dependent fashion, platelet aggregation induced by collagen (FIG 4D, upper and lower panels).

Finally, G04/Rhosin was also developed by us as a RhoA GTPase activation site inhibitor that transiently and specifically blocks RhoA activity and RhoA-mediated signaling functions (36, 37). G04 contains two aromatic rings tethered by a linker, and it binds to the surface area sandwiching Trp58 of RhoA (FIG 5A) with a submicromolar Kd and effectively inhibits GEF-catalyzed RhoA activation. In platelets, G04 specifically inhibits collagen-induced RhoA activity (FIG 5B) and RhoA-mediated cellular functions including fibrinogen-dependent platelet spreading (FIG 5C) and collagen-dependent platelet aggregation (FIG 5D). Effect by collagen or thrombin is reversible (FIG 5D, lower).

In addition to the reversibility, each inhibitor transiently mimics the effects of Cdc42, Rac1, or RhoA gene knockout in platelets, respectively, and does not show any additive effects in the respective knockout cells (data not shown), indicating their specificity and a lack of toxicity.

A combination of CASIN (10 µM), G04 (75 µM) and/or NSC23766 were used in C57B1/6 murine carboxyfluorescein-lateled platelets incubated at 0°C or 5°C for 3.5 hours. After re-warming, platelets were infused in 5 congenic mice (per group) and compared with control (room temperature stored) and refrigerated, untreated platelet group (FIG 7). While the combination of NSC23766, G04 and CASIN result in close-to-complete reversal of the survival deficiency induced by refrigerated storage, NSC23766 alone or NSC23766 in combination with CASIN or G04 did not result in significant reversal of the storage lesion of platelets (Figure 7). In a second experiment focused on the specific combinations with more activity to reverse the survival impairment associated with the use of Rho GTPase inhibitors, we analyzed the 24-hour recovery (%) and survival (hours) of platelets that had been stored in presence of several combinations of drugs (Figure 6A).

Similarly, treatment of refrigerated platelets with CASIN or G04 alone did not improve platelet 24-hour recovery (FIG 6B) or survival (FIG 6C) in vivo. However, a triple combination of CASIN, G04 and NSC23766 resulted in a significant improved recovery (FIG 6B) and survival (FIG 6C) of refrigerated platelets after infusion. Use of the same triple inhibitor combination during storage of refrigerated platelets at 5°C instead of 0°C resulted in complete reversal of the recovery and survival of refrigerated platelets in vivo (FIG 6D) as determined by multi-hit regression analysis (COSTCO software) and ANOVA test with Bonferroni correction for statistical differences. Applicant found that the 24-hour recovery of platelets refrigerated was significantly reduced (~20%) compared with the group control (stored at room temperature, p<0.01). Interestingly, the shortened recovery was completely reversed by incubation with the cocktail of three inhibitors (FIG 6D). Altogether, these data indicate that the inhibitory combination of Cdc42, Rac1, and RhoA with CASIN, NSC23766 and G04 can specifically and reversibly inhibit platelet activation and may be useful in a reversal of refrigeration storage lesion in platelets.

Finally, Rho family GTPase inhibitors can prevent human platelet storage lesion in vitro as assessed by microparticle formation and content of GpIb (CD42b) in microparticles. Applicant found that the use of inhibitors resulted in reversal of the refrigeration dependent microparticle generation (Figure 8A-B). Finally, Applicant analyzed the formation of lipid raft microdomains on the platelet membrane by differential analysis in lysates enriched in Triton-X-100 resistant membrane fragments in human platelets stored refrigerated for 3 and 7 days (Figure 9). We found a complete correlation between the results of survival of refrigerated platelets in presence of inhibitor combinations with the formation of lipid raft microdomains, suggesting that the inhibitor combination containing G04, NSC23766 and CASIN results in reversal of refrigeration-induced lipid raft formation. Interestingly, washing out G04, NSC23766 and CASIN results in restoration of the same levels of lipid rafts as found in 3 or 7 day stored platelets at room temperature (Figure 9), suggesting that the removal of these inhibitors results in platelet membrane rheological modifications similar to the ones observed in unprocessed, standard storage platelets for the same period of time (Figure 9). These data provide proof-of-concept that the long-term storage (6 days) of platelets in plasma containing Rho GTPase inhibitors is not deleterious of platelets but can further prevent their storage lesion-associated activation.

Thus, Applicant's data strongly support that reversible inhibition of multiple Rho family of GTPases by chemical inhibitors can significantly prevent refrigerated storage damage and improve platelet survival and transfusion function after refrigeration by interference with lipid raft microdomain formation, actomyosin dynamics and GPIb clustering in membrane microdomains.

Applicant further analyzed and confirmed that the reversible inhibition of multiple Rho family of GTPases by chemical inhibitors can significantly prevent refrigerated storage damage and improve platelet survival and transfusion function after refrigeration. The mechanism is believed to be through inhibiting Rho GTPase-regulated lipid raft microdomain formation, actomyosin dynamics and GPIb clustering in membrane microdomains.

To further validate our results from murine refrigerated platelet survival improvement in primates, Applicant first analyzed the survival of human pooled (n=10 donors per experiment) platelets transfused in inbred thrombocytopenic, irradiated, macrophage-depleted NSG mice. These immunodeficient mice allow the survival of human platelets for a short period of time (<24 hours) but sufficiently long to allow comparison. In this model, Applicant found that, in two independent experiments (n=4 mice per group and experiment), the incubation of platelets during storage with the inhibitors G04 (RhoA inhibitor), NSC23766 (NSC; Rac inhibitor) in absence of wash step or with a wash step at room temperature of the inhibitors used in vitro. Interestingly, Casin (Cdc42 inhibitor) did not modify the poor survival of refrigerated platelets (FIG 10A-10B). Secondly, these results were further validated by applying a combination of G04, NSC and Casin in outbred Rhesus monkey platelets that were biotinylated and autologously transfused after 7-day refrigerated storage. As seen in FIG 10C, a randomized, crossover trial where the same monkeys were transfused with control (not treated with inhibitors, control) or inhibitors (test), demonstrated that the inhibitor combination resulted in improved (positive recoveries) in the test group.

To better define the effect of RhoA inhibition and to rule out any off-target effect of G04, we analyzed platelets from wild-type (Wt) mice and from mice with interferon-induced (polyI:C) genetic deficiency of RhoA (FIG 11A). G04, and to a lesser degree NSC, were able to reduce the level of Flot-1 in the membrane lipid raft (microdomains) of refrigerated Wt platelets (FIG 11B). Interestingly, the levels of membrane-bound Flot-1 in RhoA-deficient (RhoA^{Δ/Δ}) platelets was similar to those found in G04 treated Wt refrigerated platelets (FIG 10C), and NSC does not appear to add any significant further inhibition.

Next, Applicant analyzed whether the phagocytosis of refrigerated murine platelets by activated monocytic THP-1 cells was modified by pre-treatment with the Rho family inhibitors. Pre-treatment of refrigerated platelets with G04 or the combination of G04, Casin and NSC reduce the ability to be recognized by activated THP-1 cells (FIG 12A). Use of murine RhoA genetically deficient platelets (FIG 12A, inset) were resistant to the phagocytosis induced by refrigeration and showed no response to G04 or the combination of G04, Casin and NSC (FIG 12A). These results strongly indicate that G04 blocks the ability of refrigerated platelets to present ligands susceptible of recognition by macrophage cells and this effect is exclusively dependent on RhoA activity. These results were confirmed in human platelets. Phagocytosis by THP-1 cells of refrigerated human platelets was completely prevented by storage with G04 or combination of G04, Casin and NSC even after wash out of the inhibitors at room temperature (FIG 12B).

Finally, Applicant examined whether the absence of translocation of lipid raft microdomains to the membrane (FIG 9) correlates with changes in galactosyl-transferase and sialyl-transferase activities. These activities should reduce by transfer to other substrates the galactosyl and sialyl residues of GpIb on the membrane, a crucial step in the process of refrigeration storage lesion of platelets. Applicant found that G04 or G04 in combination with Casin and NSC completely prevented the presence of galactosyl- (FIG 12C) or sialyl- (FIG 12D) transferase activities on the platelet membrane. This effect is completely reversible since washing of the inhibitors restores the transport of these enzymes to the membrane (FIG 12C-D).

Altogether, these new data strongly support the conclusion that Rho GTPase inhibitor G04 alone or G04 in combination with NSC23766 and/or Casin will prevent refrigerated storage lesion of platelets. This effect is reproduced in mouse, human and non-human primate platelets. The mechanism involves the prevention of formation of galactosyl- and sialyl-transferase rich membrane lipid rafts on refrigerated platelet membranes through a modulation of actomyesin dynamics.

All percentages and ratios are calculated by weight unless otherwise indicated.

All percentages and ratios are calculated based on the total composition unless otherwise indicated.

## Claims

1. A composition, comprising platelets and a RHOA inhibitor selected from: 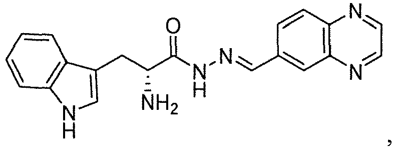 or salt thereof, 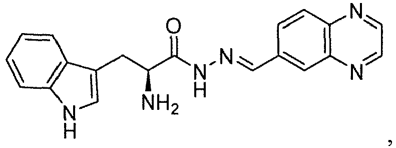 or salt thereof, or combinations thereof; and an acceptable carrier.

2. A method for storing platelets comprising forming the composition of Claim 1, and storing said composition.

3. A method of reversing platelet activation comprising forming the composition of Claim 1.

4. A method of reversing refrigeration storage lesion in platelets comprising forming the composition of Claim 1, wherein said platelets have refrigeration storage lesions.

5. A method for slowing platelet damage comprising forming the composition of Claim 1.

6. The composition according to Claim 1 or method according to any one of Claims 2-5, wherein the carrier comprises saline, phosphate buffered saline, Tris buffered saline, Hank's buffered saline, water, an electrolyte solution, or combination thereof.

7. The method according to any one of Claims 2-6, wherein the platelets undergo storage in the presence of the RHOA inhibitor at a temperature from 1 °C to 20 °C.

8. The composition according to any of Claims 1 or 6 or method according to any one of Claims 2-7, further comprising an additive selected from NaCl, KCl, CaCl₂, MgCl₂, MgSO₄, Na₃ citrate, citric acid, NaHCO₃, Na phosphate, Na acetate, Na gluconate, glucose, maltose, mannitol, D-ribose, D-glucose, Hank's solution, HEPES solution, bovine serum albumin, tick anticoagulant peptide, sterile water, a pH adjusting agent, a pH buffering agent, a tonicity adjusting agent, a stabilizer, a wetting agent, and combinations thereof.

9. The composition according to any of Claims 1, 6, 8 or method according to any one of Claims 2-8, further comprising a therapeutic agent.

10. The composition according to any of Claims 1, 6, 8, 9 or method according to any one of Claims 2-9, wherein the RHOA inhibitor is 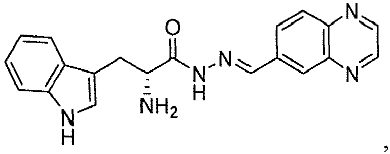 or salt thereof.

11. The composition according to any of Claims 1, 6, 8-10 or method according to any one of Claims 2-9, wherein the RHOA inhibitor is 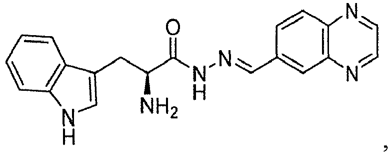 or salt thereof.

12. The composition according to any of Claims 1, 6, 8-11 or method according to any one of Claims 2-11, wherein the RHOA inhibitor is present in said carrier at a concentration of at least 10 µM.

13. The use of a composition according to any of the Claims 1, 6, 8-12 in the storage of platelets at a temperature from 1°C to 20°C.

## Patentansprüche

1. Zusammensetzung, umfassend Blutplättchen und einen RHOA-Inhibitor ausgewählt unter: 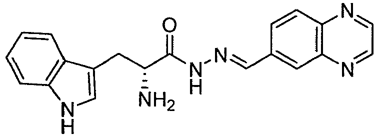 oder ein Salz davon, 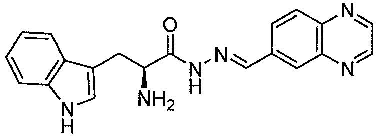 oder ein Salz davon, oder eine Kombination dieser; und ein verträglicher Träger.

2. Verfahren zum Lagern von Blutplättchen, umfassend das Bilden der Zusammensetzung nach Anspruch 1 und Lagern der Zusammensetzung.

3. Verfahren zur Umkehrung der Blutplättchenaktivierung, umfassend das Bilden der Zusammensetzung nach Anspruch 1.

4. Verfahren zur Umkehrung von Kühlungslagerungsläsionen von Blutplättchen, umfassend das Bilden der Zusammensetzung nach Anspruch 1, wobei die Blutplättchen Kühlungslagerungsläsionen aufweisen.

5. Verfahren zum Verlangsamen der Schädigung von Blutplättchen, umfassend das Bilden der Zusammensetzung nach Anspruch 1.

6. Zusammensetzung nach Anspruch 1 oder Verfahren nach einem der Ansprüche 2 bis 5, wobei der Träger eine Kochsalzlösung, eine mit Phosphat gepufferte Kochsalzlösung, eine mit Tris gepufferte Kochsalzlösung, eine mit Hank's gepufferte Kochsalzlösung, Wasser, eine Elektrolytlösung oder Kombinationen davon umfasst.

7. Verfahren nach einem der Ansprüche 2 bis 6, wobei die Blutplättchen einer Lagerung in Gegenwart des RHOA-Inhibitors bei einer Temperatur von 1 °C bis 20 °C unterzogen werden.

8. Zusammensetzung nach einem der Ansprüche 1 oder 6 oder ein Verfahren nach einem der Ansprüche 2 bis 7, weiterhin umfassend ein Additiv ausgewählt unter NaCl, KCl, CaCl₂, MgCl₂, MgSO₄, Na₃-Citrat, Zitronensäure, NaHCO₃, Na-Phosphat, Na-Acetat, Na-Gluconat, Glucose, Maltose, Mannitol, D-Ribose, D-Glucose, Hank's Lösung, HEPES-Lösung, bovinem Serumalbumin, Tick-Anitcoagulationspeptid, sterilem Wasser, einem Mittel zum Einstellen des pH-Wertes, einem Mittel zum Puffern des pH-Wertes, einem Mittel zur Einstellung der Tonizität, einem Stabilisator, einem Befeuchtungsmittel und Kombinationen davon.

9. Zusammensetzung nach einem der Ansprüche 1, 6, 8 oder Verfahren nach einem der Ansprüche 2 bis 8, weiterhin umfassend ein therapeutisches Mittel.

10. Zusammensetzung nach einem der Ansprüche 1, 6, 8, 9 oder Verfahren nach einem der Ansprüche 2 bis 9, wobei der RHOA-Inhibitor 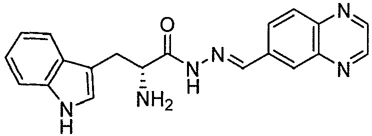 oder ein Salz davon ist.

11. Zusammensetzung nach einem der Ansprüche 1, 6, 8 bis 10 oder Verfahren nach einem der Ansprüche 2 bis 9, wobei der RHOA-Inhibitor 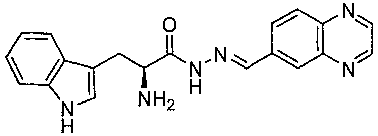 oder ein Salz davon ist.

12. Zusammensetzung nach einem der Ansprüche 1, 6, 8 bis 11 oder Verfahren nach einem der Ansprüche 2 bis 11, wobei der RHOA-Inhibitor in dem Träger in einer Konzentration von mindestens 10 µM vorliegt.

13. Verwendung der Zusammensetzung nach einem der Ansprüche 1, 6, 8 bis 12 zum Lagern von Blutplättchen bei einer Temperatur von 1 °C bis 20 °C.

## Revendications

1. Composition, comprenant des plaquettes et un inhibiteur de RHOA sélectionné parmi : 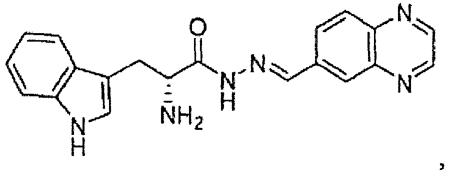 ou un sel de celui-ci, 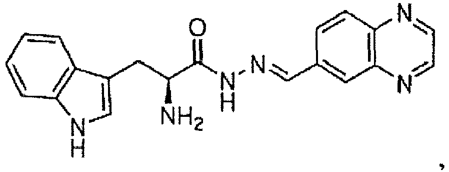 ou un sel de celui-ci, ou les combinaisons de ceux-ci ; et un support acceptable.

2. Méthode de stockage de plaquettes comprenant la formation de la composition selon la revendication 1, et le stockage de ladite composition.

3. Méthode de suppression de l'activation des plaquettes comprenant la formation de la composition selon la revendication 1.

4. Méthode de suppression d'une lésion due à un stockage par réfrigération dans des plaquettes comprenant la formation de la composition selon la revendication 1, dans laquelle lesdites plaquettes présentent des lésions dues à un stockage par réfrigération.

5. Méthode de ralentissement de l'endommagement des plaquettes comprenant la formation de la composition selon la revendication 1.

6. Composition selon la revendication 1 ou méthode selon l'une quelconque des revendications 2 à 5, dans laquelle le support comprend une solution saline, une solution saline tamponnée au phosphate, une solution saline tamponnée au Tris, une solution saline tamponnée de Hank, de l'eau, une solution d'électrolyte, ou une combinaison de celles-ci.

7. Méthode selon l'une quelconque des revendications 2 à 6, dans laquelle les plaquettes sont stockées en présence de l'inhibiteur de RHOA à une température de 1 °C à 20 °C.

8. Composition selon l'une quelconque des revendications 1 ou 6 ou méthode selon l'une quelconque des revendications 2 à 7, comprenant en outre un additif sélectionné parmi NaCl, KCl, CaCl₂, MgCl₂, MgSO₄, le citrate de Na₃, l'acide citrique, NaHCO₃, le phosphate de Na, l'acétate de Na, le gluconate de Na, le glucose, le maltose, le mannitol, le D-ribose, le D-glucose, une solution de Hank, une solution HEPES, une sérumalbumine bovine, un peptide anticoagulant de tique, une eau stérile, un agent d'ajustement de pH, un agent tampon de pH, un agent d'ajustement de tonicité, un agent de stabilisation, un agent mouillant, et les combinaisons de ceux-ci.

9. Composition selon l'une quelconque des revendications 1, 6, 8 ou méthode selon l'une quelconque des revendications 2 à 8, comprenant en outre un agent thérapeutique.

10. Composition selon l'une quelconque des revendications 1, 6, 8, 9 ou méthode selon l'une quelconque des revendications 2 à 9, dans laquelle l'inhibiteur de RHOA est 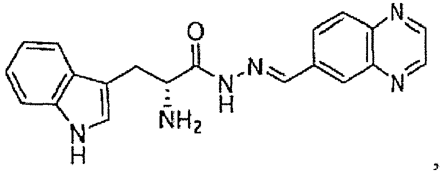 ou un sel de celui-ci.

11. Composition selon l'une quelconque des revendications 1, 6, 8 à 10 ou méthode selon l'une quelconque des revendications 2 à 9, dans laquelle l'inhibiteur de RHOA est 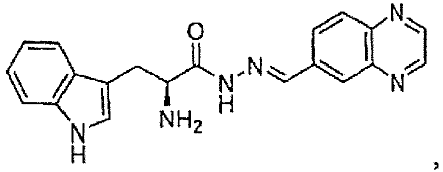 ou un sel de celui-ci.

12. Composition selon l'une quelconque des revendications 1, 6, 8 à 11 ou méthode selon l'une quelconque des revendications 2 à 11, dans laquelle l'inhibiteur de RHOA est présent dans ledit support à une concentration d'au moins 10 µM.

13. Utilisation d'une composition selon l'une quelconque des revendications 1, 6, 8 à 12 lors du stockage de plaquettes à une température de 1 °C à 20 °C.
